(19) **Europäisches Patentamt / European Patent Office / Office européen des brevets**

(11) **EP 4 395 641 B1**

(12) **EUROPEAN PATENT SPECIFICATION**

(45) Date of publication and mention of the grant of the patent:
**18.12.2024 Bulletin 2024/51**

(21) Application number: **22764475.4**

(22) Date of filing: **31.08.2022**

(51) International Patent Classification (IPC):
*A61B 5/12* (2006.01)       *A61B 5/378* (2021.01)
*G16H 50/20* (2018.01)      *A61B 3/10* (2006.01)
*G16H 40/63* (2018.01)      *A61B 5/38* (2021.01)

(52) Cooperative Patent Classification (CPC):
**A61B 5/125; A61B 5/378; A61B 5/38; G16H 40/63;**
A61B 5/377; G16H 40/67

(86) International application number:
**PCT/NL2022/050495**

(87) International publication number:
**WO 2023/033647 (09.03.2023 Gazette 2023/10)**

(54) **DETERMINING A PERSON'S SENSORY CAPABILITY BASED ON STIMULUS RESPONSE AMPLITUDE WEIGHTS**

BESTIMMUNG DER SENSORISCHEN FÄHIGKEIT EINER PERSON AUF DER BASIS VON STIMULUSREAKTIONSAMPLITUDENGEWICHTEN

DÉTERMINATION DE LA CAPACITÉ SENSORIELLE D'UNE PERSONNE SUR LA BASE DE PONDÉRATIONS D'AMPLITUDE DE RÉPONSE À UN STIMULUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2021 NL 2029113**

(43) Date of publication of application:
**10.07.2024 Bulletin 2024/28**

(73) Proprietor: **Mindaffect B.V.**
**6718 TK Ede (NL)**

(72) Inventors:
• **FARQUHAR, Jason David Robert**
**6718 TK Ede (NL)**
• **VAN KESTEREN, Mark Wilhelmus Theodorus**
**6718 TK Ede (NL)**
• **PORTOLES MARIN, Oscar**
**6718 TK Ede (NL)**
• **KODERMAN, Eva**
**6718 TK Ede (NL)**

(74) Representative: **De Vries & Metman**
**Overschiestraat 180**
**1062 XK Amsterdam (NL)**

(56) References cited:
CN-A- 106 456 039       GB-A- 2 559 984
US-A- 4 846 567         US-A- 6 086 206
US-A1- 2017 202 476     US-A1- 2019 255 350
US-B2- 6 688 746

• ELBERLING CLAUS ET AL: "Auditory brainstem responses to a chirp stimulus designed from derived-band latencies in normal-hearing subjects", THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 124, no. 5, 1 November 2008 (2008-11-01), pages 3022 - 3037, XP012120555, ISSN: 0001-4966, DOI: 10.1121/1.2990709

**Description**

FIELD OF THE INVENTION

The invention relates to a system for determining a person's sensory capabilities and/or the psychological and/or neurological state of the person.

BACKGROUND OF THE INVENTION

In several applications, the determination of a person's sensory capability is a crucial step. Examples of such applications are configuring a hearing aid and determining an eyeglass prescription. Normally, a plurality of stimuli is presented. A person may then be asked to assess the presented stimuli. In order to determine the person's sensory capability more objectively, brain wave signals (e.g., EEG data or other electrophysiological data) may be analyzed instead.

When an external stimulus is processed by the brain, it evokes a unique response which encodes information about the stimulus dependent processing which has taken place. Using external electrophysiological sensors, such as EEG or MEG, or internal ones, such as ECoG, some properties of this evoked response can be measured. Subsequent analysis of the measured Evoked Potential (EP) can then be used to infer certain properties of the stimulus as perceived and processed by the brain, which may be useful for monitoring or diagnosis of the brain's sensory processing capabilities.

The most common technique to estimate a stimulus response is to compute a simple response average for each electrophysiological sensor time-locked to the stimulus onset time. The response estimate computed in this way is commonly termed an Event Related Potential (ERP).

For example, an auditory steady state response (ASSR) is an auditory evoked potential (AEP) that can be used to objectively estimate hearing sensitivity in individuals with normal hearing sensitivity and with various degrees and configurations of sensorineural hearing loss (SNHL). Audio-metric testing systems normally present stimuli with different stimulus parameter combinations, specifically audio intensity and tone.

US 6,602,202 B2 discloses testing of auditory performance using periodically presented auditory chirp stimuli with varying central-tone and auditory intensity and measuring the EEG response - the above-mentioned ASSR. In addition, Amplitude Modulation and Frequency Modulation of the chirps is used to enhance the strength of the generated ASSR. The results are then analyzed using a noise-reducing weighted averaging technique to obtain estimates of the amplitude and latency of the ASSR for each tone and intensity combination.

The paper "Auditory Steady-State Responses" by Peggy Korczak et al., Journal of the American Academy of Audiology 23(3): 146-70, March 2012, describes how existing ASSR based audio-metric testing systems use a clever design to rapidly test these stimulus parameter combinations. This design has four key properties:

  1. The stimuli are presented to the user in a series of steps over audio intensity, starting from low audio intensity and sequentially increasing to max audio intensity.
  2. Within an audio intensity, tones are presented at a fixed interval, e.g., every 1/30 seconds. This means that any brain response for this tone will also be generated every 1/30 seconds leading to an increased power in the EEG at 30Hz irrespective of the exact shape of an individual tone's brain response.
  3. Multiple tones are presented at the same time, but with slightly different inter tone intervals. For example, the 500Hz tone is presented every 1/30s, the 1000Hz every 1/27 seconds, the 2000Hz every 1/33 seconds and the 4000Hz every 1/37 seconds. As each tone has a unique interval, they generate a power increase with a unique frequency in the EEG. In this way all 4 tones can be tested in a single step, and even though the individual tone responses all overlap in the EEG the individual response strength can still be extracted by selecting the appropriate response frequency.
  4. Individual tones are not a single pure frequency, but Chirps, which span a range of frequencies around the central tone, where the chirps are specifically designed such that the evoked neural response is maximized.

The paper "Comparison of auditory brainstem response and auditory steady state response audiometry by evaluating the hearing thresholds obtained in children with different severity of hearing loss" by Muhammad Azeem Aslam et al., Pakistan Journal of Medical Sciences, 2019 Mar-Apr; 35(2): 320-324, reports that this design allows determination of audio-metric curves in approximately 15 minutes. The longer this determination takes, the more time is taken up from the subject and from the worker that conducts the test. It is therefore beneficial to reduce the duration of the test.

The paper "Auditory brainstem responses to a chirp stimulus designed from derived-band latencies in normal-hearing subjects" by Claus Elberling et al., J. Acoust. Soc. Am., November 2008, 124(5): 3022-3037, describes a system for determining a person's sensory capabilities based on brain waves such as the auditory brain-stem response (ABR).

SUMMARY OF THE INVENTION

It is an object of the invention to provide a system, which can determine a person's sensory capability with a relatively short test.

In a first aspect, a computer-implemented method of determining a person's sensory capabilities comprises obtaining one or more brain wave signals, the one more brain wave signals being measured on the person by a plurality of electrophysiological sensors, obtaining stimulus data representing a plurality of sensory stimuli presented over time with a plurality of levels, each of the stimuli being associated in the stimulus data with a level of the plurality of levels, determining a mathematical model in which the one or more brain wave signals are equal to an expression which comprises a sum of each of a plurality of spatial patterns multiplied with a factor representing activity of a corresponding neural source, the factor comprising a convolution of the stimulus data with the plurality of levels and stimulus responses for each of the neural sources, the stimulus responses being weighted with a stimulus response amplitude weight per level of the plurality of levels, estimating the plurality of spatial patterns, the stimulus responses, and the stimulus response amplitude weights in the mathematical model, and determining the person's sensory capabilities based on the stimulus response amplitude weights. The method may be performed by software running on a programmable device. This software may be provided as a computer program product. The sensory stimuli may comprise audio and/or visual and/or tactile and/or pain stimuli, for example.

Without this mathematical model, when using conventional techniques, the activity from a single neural source is spatially smeared out over multiple electrodes, the response at each channel (each channel being associated with an electrode) represents a summation of activity from many brain regions (i.e. neural sources), the temporal response for a single stimulus gets temporally superimposed with other overlapping earlier and later stimulus responses, thereby introducing additional noise in the stimulus response estimation, and the response for each stimulus is estimated in isolation, thereby ignoring any shared structure between responses for different stimuli (such as when the response has a common shape but stimulus-dependent amplitudes). This makes it necessary to estimate a greater number of stimulus responses, specifically, #channels * #time-points * #stimuli, requiring more data and reducing estimation efficiency, and practically requiring more user time to complete the test.

The above-described mathematical model results in significantly fewer parameters (i.e., #channels + #time-points + #stimuli). For example, a 64 channel (i.e. with 64 electrodes) recording sampled at 200Hz with a stimulus response duration of .6s for 4 stimuli, requires estimation of 64*(.6*200)*4 = 30720 parameters for the conventional mathematical model, but only 64+(.6*200)+4 = 188 parameters for the above-described mathematical model, so just over 100 times fewer parameters. This reduction in parameters may result in a reduction of the data collection time required to reach a particular model quality, possibly in a similar significant reduction in data collection time. A person's sensory capability may therefore by determined with a relatively short test.

This level-dependent response amplitude model is appropriate for many problems where a stimulus response has a similar shape but varying amplitude as stimulus characteristics are varied.

In hearing testing, the EP amplitude normally reduces with reducing audio intensity and drops to zero response below the user's detection threshold. Further, as the user's detection threshold is dependent on the stimulus tone, the amplitude normally varies both with tone and audio intensity. The EP amplitude may be proportional to the stimulus level or to a component of the stimulus level, e.g., audio intensity, but may also vary in other ways.

Amongst others, US 6,602,202 B2 does not disclose 1) using non-periodic presentation of the auditory chirp stimuli; 2) fitting a model to the measured EEG which estimates the full auditory evoked potential; and 3) fitting a model for the per stimulus responses which directly model how the response amplitude varies with changing tone and/or intensity. These differences represent an advance in requiring less subject measurement time to produce a more complete estimate of the Auditory evoked potential.

In vision testing, the EP amplitude of a fixed angular-size stimulus reduces as one moves further from the center of the visual field (due to cortical magnification). Further, if the user has localized visual deficits (such as caused by Glaucoma, or ROP, or retinal damage), localized amplitude reductions may be observed at the affected locations in the visual field. In addition, the amplitude of the EP of a fixed angular-size stimulus decreases as the color or luminance contrast in the visual stimuli reduces. Complex visual stimuli such as faces with decreasing degree of familiarity or increasing degree of deformation also reduce the amplitude of the EP. As this level-dependent response amplitude model has many fewer parameters than the full model, it not only gives much more interpretable output but can be reliably estimated using many fewer data points.

In tactile testing, the EP amplitude for tactile stimulation (such as with a braille stimulator) at a fixed body location reduces as the stimulus amplitude decreases. Similarly, in pain threshold testing, the EP amplitude (at a fixed location) reduces as the pain stimulus amplitude decreases.

The method may further comprise configuring a hearing aid based on the person's sensory capabilities. Determining the person's sensory capabilities may comprise determining an audio metric threshold, for example. For example, the estimated stimulus response amplitude weights may be used as input for a machine learning system to optimize the hearing aid settings to match the user's auditory capabilities and minimize the required hearing effort. In this way, the hearing aid can automatically fit itself to each individual user to maximize their satisfaction over time. Similarly, the method may be used to automatically optimize an audio speaker system, to optimize the audio parameters to compensate for acoustic transmission properties of the space in addition to the hearing abilities of the listener.

The method may further comprise providing real-time feedback to the user to allow them to enhance or suppress their sensory capabilities using neuro-feedback. For example, to enhance a user's sensory capabilities. the currently estimated stimulus-response amplitude weights may be used as an input for a feedback system which rewards the user when the weights increase (and thus when their sensory capabilities are enhanced). This may be useful when learning or training new sensory perception skills, such as when learning a second language. Alternatively, a user may be trained to for reduced sensitivity to a particular stimulus by rewarding decreases in stimulus-response amplitude weight (thereby rewarding the user when their sensory capabilities are suppressed). This may be particularly useful for users with chronic conditions, such as non-treatable chronic pain, or tinnitus.

The method may comprise use of an objective measure for calibration of subjective measures -- such as auditory testing using button presses.

The spatial patterns, the stimulus responses, and the stimulus response amplitude weights may be estimated in the mathematical model by using Alternating Least Squares (ALS) or iterative Canonical-Correlation Analysis (CCA), for example.

The expression typically further comprises a factor representing unmodelled signal and noise. The method may further comprise measuring the one or more brain wave signals. The stimulus data may distinguish only between the periods during which the stimuli are on and the periods during which the stimuli are off or may distinguish multiple event types. The former provides good results when performing auditory testing. Thus, multiple event types do not need to be used in this case. Each of the levels may represent an audio intensity or a position in the user's visual field. For example, the levels may have been determined for a plurality of audio intensities and a plurality of tones, each of the levels representing a different combination of audio intensity and tone. The latter levels are beneficial for auditory testing.

Each of the tones may have an own unique pseudo-random sequence, each of the pseudo-random noise sequences specifying which audio intensity of the tone is to be played at a particular instant in time. To minimize analysis time this pseudo-random sequence may be maximally uncorrelated between tones and intensity levels. Examples of pseudo-random sequences with this property include (but are not limited to), multi-level gold codes, multi-level m-sequences. This design makes it possible to simply extract the individual response shape for a given level from the measured stimulus response by cross-correlating the measured stimulus response with that level's stimulus sequence as, due to the uncorrelated nature of the sequences, any interference from other levels automatically cancels out to zero. All tones pseudo-random sequences may be presented at the same time to reduce data collection time.

A plurality of events of different event types may be distinguished in each of the stimuli, the stimulus data may represent the sensory stimuli presented over time for each of the levels and for each of the event types, the stimulus responses may be determined for each of the neural sources and each of the event types, and the stimulus response amplitude weights may be independent of the event types. For example, one of the event types may represent an onset moment of the stimuli and/or one of the event types may represent an offset moment of the stimuli. This provides good results when performing visual testing.

Different event types than the ones described above may be used. For example, the tone of an audio stimulus may be an event type instead of or in addition to being represented in the level of the audio stimulus.

The stimulus response amplitude weights may be constrained. For example, the stimulus response amplitude weights may be constrained to follow a smooth function and/or a psychometric function with a sigmoid-like shape. This may allow the model parameters to be estimated with less data collection time. For example, weight configurations which are not preferred may be penalized. Furthermore, this may be used to implement active learning.

The EP response shape may be constrained. For example, the EP response may be required to be 0 at stimulus onset-time -- as, when randomly presented in time, the brain cannot respond instantaneously to an incoming stimulus due to transmission and processing lags. As another example, it may be known that a particular stimulus response has a particular shape, such as the N100, P200 shape of a visual evoked response, so the EP response may be constrained to fit this response shape. Constraining the EP response shape in this way may allow the model parameters to be estimated with less data collection time. For example, EP response shapes which are not preferred may be penalized.

The method may further comprise presenting the sensory stimuli, e.g. audio and/or visual and/or tactile and/or pain stimuli. The method may further comprise determining a further plurality of levels based on the stimulus response amplitude weights, presenting a further plurality of sensory stimuli with the further plurality of levels, obtaining one or more further brain wave signals, the one more further brain wave signals being measured of the person by the plurality of electrophysiological sensors, obtaining further stimulus data representing the further sensory stimuli presented, each of the further stimuli being associated with a level of the further plurality of levels in the further stimulus data, determining a further mathematical model in which the one or more further brain wave signals are equal to an expression which comprises an sum of each of a plurality of further spatial patterns applied to a further factor representing activity of a corresponding neural source, the further factor comprising a convolution of the further stimulus data for the plurality of levels and further stimulus responses for each of the neural sources, the stimulus responses being weighted with a further stimulus response amplitude weight per level of the further plurality of levels, and estimating the further spatial patterns, the further stimulus responses, and the further stimulus response amplitude weights in the further mathematical model, and wherein the person's sensory capabilities are determined based on the stimulus response amplitude weights by determining the person's sensory capabilities determined based on the further stimulus response amplitude weights.

This active learning may further reduce the total testing time, e.g., by focusing testing examples near the audiometric threshold. For example, stimuli which maximally reduce an error in a parametric model of the weights may be adaptively presented to the user.

In a second aspect, a system for determining a person's sensory capabilities comprises at least one processor configured to obtain one or more brain wave signals, the one more brain wave signals being measured on the person by a plurality of electrophysiological sensors, obtain stimulus data representing a plurality of sensory stimuli presented over time with a plurality of levels, each of the stimuli being associated in the stimulus data with a level of the plurality of levels, determine a mathematical model in which the one or more brain wave signals are equal to an expression which comprises a sum of each of a plurality of spatial patterns multiplied with a factor representing activity of a corresponding neural source, the factor comprising a convolution of the stimulus data with the plurality of levels and stimulus responses for each of the neural sources, the stimulus responses being weighted with a stimulus response amplitude weight per level of the plurality of levels, estimate the plurality of spatial patterns, the stimulus responses, and the stimulus response amplitude weights in the mathematical model, and determine the person's sensory capabilities based on the stimulus response amplitude weights.

In a third aspect, a computer-implemented method of presenting a plurality of audio stimuli comprises creating a unique pseudo-random sequence for each tone of a plurality of tones, each of the pseudo-random noise sequences specifying which of a plurality of audio intensities of the tone is to be played at a particular instant in time and being maximally uncorrelated between said tones and said audio intensities (e.g. being a gold code), and presenting the plurality of audio stimuli. The pseudo-random sequences may all be presented at a same time.

In a fourth aspect, a system for presenting a plurality of audio stimuli comprises at least one processor configured to create a unique pseudo-random sequence for each tone of a plurality of tones, each of the pseudo-random noise sequences specifying which of a plurality of audio intensities of the tone is to be played at a particular instant in time and being maximally uncorrelated between said tones and said audio intensities (e.g. being a gold code), and present the plurality of audio stimuli. The at least one processor may be configured to present all pseudo-random noise sequences at a same time.

In a fifth aspect, a system for determining a person's sensory capabilities and/or a psychological and/or neurological state of the person comprises at least one processor configured to obtain one or more brain wave signals, the one more brain wave signals being measured on the person by a plurality of electrophysiological sensors, obtain stimulus data representing a plurality of sensory stimuli presented over time with a plurality of levels, the stimulus data lasting for a plurality of time points, each of the stimuli being associated in the stimulus data with a level of the plurality of levels and lasting for a subset of the plurality of time points, determine a mathematical model in which the one or more brain wave signals are equal to an expression which comprises a sum of each of a plurality of spatial patterns multiplied with a factor representing activity of a corresponding underlying neural source, the factor comprising a convolution of the stimulus data and isolated stimulus responses for each of the neural sources, the isolated stimulus responses being weighted with a stimulus response amplitude weight per level of the plurality of levels, each of the spatial patterns being indicative of a spatial location of the corresponding underlying neural source, estimate the plurality of spatial patterns, the stimulus responses, and the stimulus response amplitude weights in the mathematical model, and determine the person's sensory capabilities and/or the psychological and/or neurological state of the person based on the stimulus response amplitude weights.

The sensory stimuli may comprise audio and/or visual and/or tactile and/or pain stimuli, for example. Determining the psychological and/or neurological state of the person may comprise determining the cognitive development of a child, a psychological disorder of the person, and/or a neurological disorder of the person, for example.

The stimulus data may indicate the periods during which the stimuli are on. A plurality of events of different event types may be distinguished in each of the stimuli, the stimulus data may represent the sensory stimuli presented over time for each of the levels and for each of the event types, the isolated stimulus responses may be determined for each of the neural sources and each of the event types, and the stimulus response amplitude weights may be independent of the event types.

One of the event types may represent an onset moment of the stimuli and/or one of the event types may represent an offset moment of the stimuli. The expression may further comprise a factor representing unmodelled signal and noise.

Each of the levels may represent an audio intensity, a position in the user's visual field, a degree of contrast in luminance and/or color, a visual spatial resolution, a degree of familiarity with a complex visual stimulus, a degree of deformation of a complex auditory stimulus, or a degree of deformation of a complex visual stimulus.

The levels may be determined for a plurality of audio intensities and a plurality of tones, each of the levels representing a different combination of audio intensity and tone. The levels may be determined for a plurality of degrees of contrast in luminance and/or color and a plurality of positions in the user's visual field, each of the levels representing a different combination of degree of contrast in luminance and/or color and location in the user's visual field.

The levels may be determined for a plurality of degrees of color contrast and a plurality of degrees of luminance contrast at a single location in the user's visual field, each of the levels representing a different combination of degree of color contrast and degree of luminance contrast. The levels may be determined for a plurality of degrees of contrast in color and/or luminance and a plurality of visual spatial resolutions, each of the levels representing a different combination of degree of contrast in color and/or luminance and visual spatial resolution. The complex visual stimulus may comprise an image of a face and/or the complex auditory stimulus comprises a phonetic sound.

The at least one processor may be configured to create a unique pseudo-random sequence for each of a plurality of sensory stimulus features, each of the pseudo-random sequences specifying which of the plurality of levels of the corresponding sensory stimulus feature is to be presented at a particular instant in time, and present the plurality of sensory stimulus features at the plurality of levels as specified by the pseudo-random sequences.

The plurality of sensory stimulus features may comprise a plurality of tones, the plurality of levels may comprise a plurality of audio intensities, and each of the pseudo-random sequences may specify which of the plurality of audio intensities of the corresponding tone is to be played at a particular instant in time.

The plurality of sensory stimulus features may comprise a plurality of visual stimulus features and each of the pseudo-random sequences may specify at which particular location of the user's visual field and at which particular instant in time the corresponding visual stimulus feature is to be presented.

The at least one processor may be configured to measure the one or more brain wave signals and/or configure a hearing aid and/or a sight correction aid based on the person's sensory processing capabilities. Alternatively or additionally, a treatment plan may be determined based on the person's sensory processing capabilities and/or the psychological and/or neurological state of the person. The at least one processor may be configured to determine the person's sensory capabilities by determining an audiometric threshold, a contrast sensitivity threshold, and/or a visual acuity threshold.

The stimulus response amplitude weights may be constrained. For example, the stimulus response amplitude weights may be constrained to follow a smooth function and/or a psychometric function with a sigmoid-like shape.

The at least one processor may be configured to use re-sampling to determine a model parameter confidence interval of the stimulus response amplitude weights to statistically infer differences between the distributions of the stimulus response amplitude weights after re-sampling. The at least one processor may be configured to determine a measure of goodness-of-fit for each of a plurality of mathematical models, the plurality of mathematical models differing in used sensory stimuli and/or in used constraints on parameters of the mathematical model, and select one of the plurality of models based on the determined measures of goodness-of-fit of the model.

In a sixth aspect, a system for presenting a plurality of sensory stimuli features at a plurality of levels, the plurality of sensory stimuli features comprising a plurality of audio stimulus features and/or a plurality of visual stimulus features, the system comprising at least one processor configured to create a unique pseudo-random sequence for each of the sensory stimuli features, each of the pseudo-random sequences specifying which of the plurality of levels of the corresponding sensory stimulus feature is to be presented at a particular instant in time, and present the sensory stimulus features at the plurality of levels as specified by the pseudo-random sequences.

Each of the pseudo-random sequences may be maximally uncorrelated between different levels of the corresponding sensory stimulus feature and/or the different pseudo-random sequences may be maximally uncorrelated between different sensory stimulus features. The plurality of audio stimulus features may comprise a plurality of tones, the plurality of levels may comprise a plurality of audio intensities, and each of the pseudo-random sequences may specify which of the plurality of audio intensities of the corresponding tone is to be played at a particular instant in time.

The plurality of sensory stimulus features may comprise a plurality of visual stimulus features and each of the pseudo-random sequences may specify at which particular location of the user's visual field and at which particular instant in time the corresponding visual stimulus feature is to be presented. For example, each of a plurality of degrees of luminance and/or color contrast and/or each of a plurality of spatial resolutions may have an own unique pseudo-random sequence.

Moreover, a computer program for carrying out the methods described herein, as well as a non-transitory computer readable storage-medium storing the computer program are provided. A computer program may, for example, be downloaded by or uploaded to an existing device or be stored upon manufacturing of these systems.

A non-transitory computer-readable storage medium stores at least a first software code portion, the first software code portion, when executed or processed by a computer, being configured to perform executable operations for determining a person's sensory capabilities.

These executable operations comprise obtaining one or more brain wave signals, the one more brain wave signals being measured on the person by a plurality of electrophysiological sensors, obtaining stimulus data representing a plurality of sensory stimuli presented over time with a plurality of levels, each of the stimuli being associated in the stimulus data with a level of the plurality of levels, determining a mathematical model in which the one or more brain wave signals are equal to an expression which comprises a sum of each of a plurality of spatial patterns multiplied with a factor representing activity of a corresponding neural source, the factor comprising a convolution of the stimulus data with the plurality of levels and stimulus responses for each of the neural sources, the stimulus responses being weighted with a stimulus response amplitude weight per level of the plurality of levels, estimating the plurality of spatial patterns, the stimulus responses, and the stimulus response amplitude weights in the mathematical model, and determining the person's sensory capabilities based on the stimulus response amplitude weights.

A non-transitory computer-readable storage medium stores at least a second software code portion, the second software code portion, when executed or processed by a computer, being configured to perform executable operations for presenting sensory stimuli.

These executable operations comprise creating a unique pseudo-random sequence for each tone of a plurality of tones, each of the pseudo-random noise sequences specifying which of a plurality of audio intensities of the tone is to be played at a particular instant in time and being maximally uncorrelated between said tones and said audio intensities, and presenting the plurality of audio stimuli.

As will be appreciated by one skilled in the art, aspects of the present disclosure may be embodied as a device, a method or a computer program product.

Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "circuit", "module" or "system." Functions described in this disclosure may be implemented as an algorithm executed by a processor/microprocessor of a computer. Furthermore, aspects of the present disclosure may take the form of a computer program product embodied in one or more computer readable medium(s) having computer readable program code embodied, e.g., stored, thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples of a computer readable storage medium may include, but are not limited to, the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present disclosure, a computer readable storage medium may be any tangible medium that can contain, or store, a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber, cable, RF, etc., or any suitable combination of the foregoing. Computer program code for carrying out operations for aspects of the present disclosure may be written in any combination of one or more programming languages, including an object oriented programming language such as Java(TM), Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present disclosure are described below with reference to flowchart illustrations and/or block diagrams of methods, apparatus (systems), and computer program products according to embodiments of the present disclosure.

It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or a central processing unit (CPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the figures illustrate the architecture, functionality, and operation of possible implementations of devices, methods and computer program products according to various embodiments of the present disclosure.

In this regard, each block in the flowchart or block diagrams may represent a module, segment, or portion of code, which comprises one or more executable instructions for implementing the specified logical function(s).

It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

## BRIEF DESCRIPTION OF THE DRAWINGS

These and other aspects of the invention are apparent from and will be further elucidated, by way of example, with reference to the drawings, in which:

Fig. 1 is a flow diagram of a first embodiment of the method;
Fig. 2 is a flow diagram of a second embodiment of the method;
Fig. 3 shows a schematic representation of an embodiment of the mathematical model;
Fig. 4 shows examples of estimated model parameters;
Fig. 5 is a block diagram of an embodiment of the system;
Fig. 6 shows a first set of functions/equations used in certain implementations of the method;
Fig. 7 shows a second set of functions/equations used in certain implementations of the method;
Fig. 8 is a flow diagram of a third embodiment of the method;
Fig. 9 shows a third set of functions/equations used in certain implementations of the method; and
Fig. 10 is a block diagram of an exemplary data processing system for performing the method of the invention.

Corresponding elements in the drawings are denoted by the same reference numeral.

## DETAILED DESCRIPTION OF THE DRAWINGS

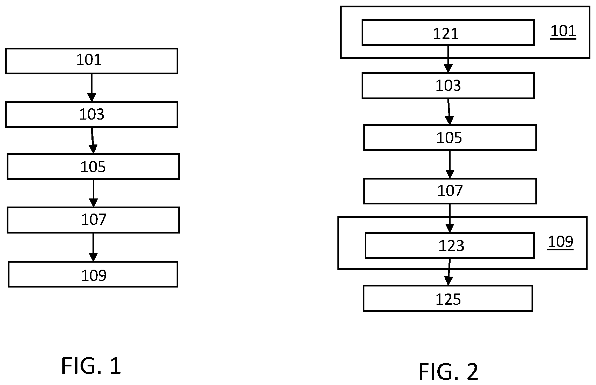

A first embodiment of the computer-implemented method of determining a person's sensory capabilities is shown in Fig. 1. A step 101 comprises obtaining one or more brain wave signals. The one more brain wave signals are measured on the person by a plurality of electrophysiological sensors. A step 103 comprises obtaining stimulus data representing a plurality of sensory stimuli presented over time with a plurality of levels. Each of the stimuli are associated in the stimulus data with a level of the plurality of levels. The sensory stimuli may comprise audio and/or visual and/or tactile and/or pain stimuli, for example.

A step 105 comprises determining a mathematical model in which the one or more brain wave signals (obtained in step 101) are equal to an expression which comprises a sum of each of a plurality of spatial patterns multiplied with a factor representing activity of a corresponding neural source. The factor comprises a convolution of the stimulus data (obtained in step 103) with the plurality of levels and stimulus responses for each of the neural sources. The stimulus responses are weighted with a stimulus response amplitude weight per level of the plurality of levels. This mathematical model is also referred to as the level-dependent response amplitude model in this patent specification.

A step 107 comprises estimating the plurality of spatial patterns, the stimulus responses, and the stimulus response amplitude weights in the mathematical model. A step 109 comprises determining the person's sensory capabilities and/or the psychological and/or neurological state of the person based on the stimulus response amplitude weights estimated in step 107. Optionally, the person's sensory capabilities are further determined based on the stimulus responses and/or the spatial patterns.

At the simplest level, the simple presence or absence of a response indicates that a presented stimulus has been perceived by the brain. This can be used for determining the person's sensory capabilities, for example can they see this stimulus, or in more detail, can they see this stimulus at this location? Or can they see this stimulus at this location with the color? Or can they hear this beep, or can they hear this beep at this volume with this frequency.

At the next level of complexity, a check may be performed whether there is a detectable difference in response when two different stimuli are presented. This can give more information about the sensitive and resolution of the person's specific sensory capabilities. For example, can the user see the difference between two small symbols (to test visual acuity), or in more detail, can they see this difference at this location, in this color (to test visual acuity at a specific location in the visual field), or can they hear the difference between these two beeps (to test tone sensitivity)?

At the next level of complexity, the specific properties of the measured brain response may be analyzed, as this can contain information about how the stimulus has been processed by the complete sensory processing pathway, with particular properties being associated with particular sensory or neural effects. For example, does one stimulus produce a delayed response compared to a similar stimulus? (this can reflect the cognitive difficulty of processing the stimulus for this user.) Is the evoked stimulus response delayed compared to the general population (this can reflect issues with the transmission of the stimulus along the sensory neurons). Is the amplitude of the response lower compared to a similar stimulus? (this can indicate the perceived intensity of the stimulus.) Or is the shape and location of the stimulus response different compared to normative values? (this can indicate particular issues in processing the stimulus.)

A second embodiment of the computer-implemented method of determining a person's sensory capabilities is shown in Fig. 2. The second embodiment of Fig. 2 is an extension of the first embodiment of Fig. 1. In the embodiment of Fig. 2, step 101 is implemented by a step 121. Step 101 comprises obtaining one or more brain wave signals. In step 121, the one or more brain wave signals are not obtained from a server but obtained by measuring the one or more brain wave signals.

In the embodiment of Fig. 2, step 109 is implemented by a step 123. Step 123 comprises determining an audio metric threshold based on the stimulus response amplitude weights. For example, if the weight for a certain level is 0, it may be assumed that the sound is not perceived by the brain, and hence it is below that person's audio metric threshold.

Alternatively, the weights may be used as bias with respect to results of other audio-metric estimation methods, which would typically be calibrated on the basis of calibration experiments, to provide clinicians with the type of result that they are used to and to build confidence in the new method. A step 125 is performed after step 123. Step 125 comprises configuring a hearing aid based on the person's sensory capabilities determined in step 109.

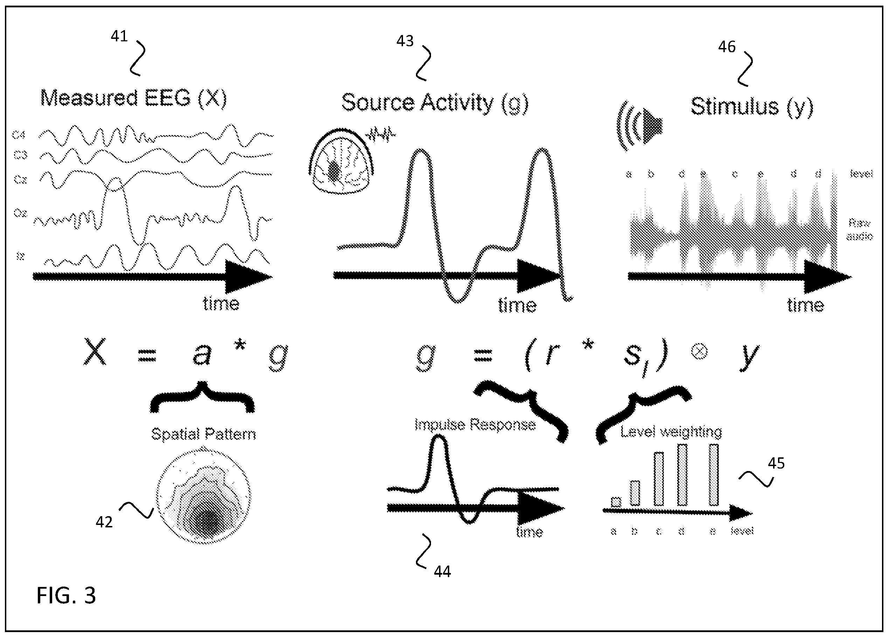

Fig. 3 shows a schematic representation of an embodiment of the mathematical model. Fig. 3 shows how the source activity 43 (g) at each neural source arises as the linear temporal convolution of the stimulus/impulse response (r), weighted with level-dependent weights 45 (s), and the stimulus data/sequence 46 (y). The measured brain wave signal 41 (X) is the further transformation of the source activity 43 (g) at each neural source with a neural source-specific spatial pattern 42 (a). The stimulus data may indicate the periods during which the stimuli are on, for example. Such stimulus data is beneficial for auditory testing, for example.

When using this mathematical method for determining a person's auditory capabilities, the levels have preferably been determined for a plurality of audio intensities and a plurality of tones, each of the levels representing a different combination of audio intensity and tone. For the sake of simplicity, only audio intensity is indicated for the levels shown in Fig. 3. The audiometric threshold can be determined based on the level-dependent weights, because levels above the audiometric for a particular tone will evoke a response which increases roughly linearly with the audio intensity and levels below the audiometric threshold will not evoke any response (and hence have weight 0). The shape of the stimulus response is the same for all levels.

The schematic representation of Fig. 3 illustrates an example in which there is only a single event type and a single neural source, The subscripts k and e (which will be described later) have therefore been omitted from Fig. 3 for clarity. In the example of Fig. 3, levels correspond to discretizations of audio intensity and are denoted by the letters a, b, c, d, and e.

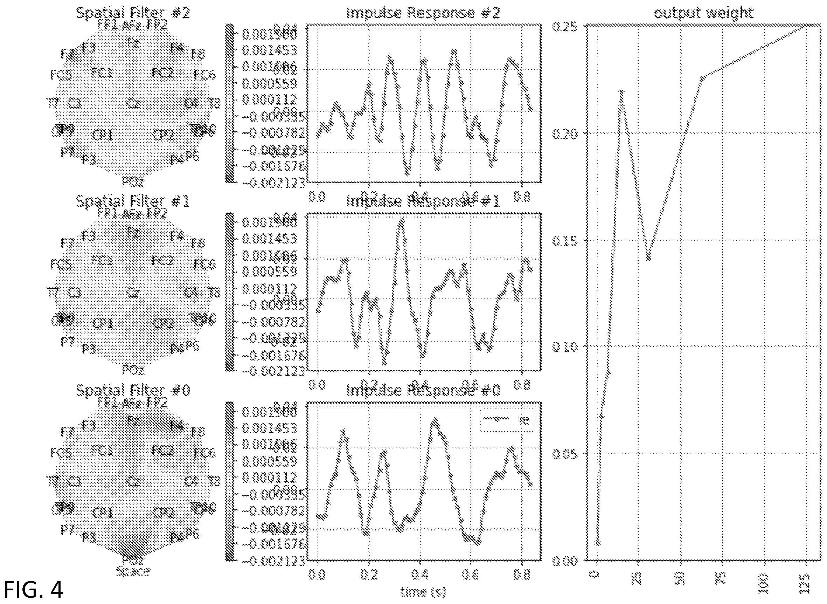

Fig. 4 shows an example of estimated model parameters. The left plots shows the estimated spatial patterns/filters for three neural sources. The middle plots show the estimated stimulus/impulse response over time for a tone presented at time 0. The right plots shows the estimated level-dependent weighting over all source locations. Source 0 appears to be a relatively slow fronto-central response. Source 1 is also mainly fronto-central with a clear peak response about 300ms post stimulus. Source 2 is more scattered with what looks like a more oscillatory response. The weighting over these sources shows a clear level-dependance, with all but the lowest-level evoking a similar strong response.

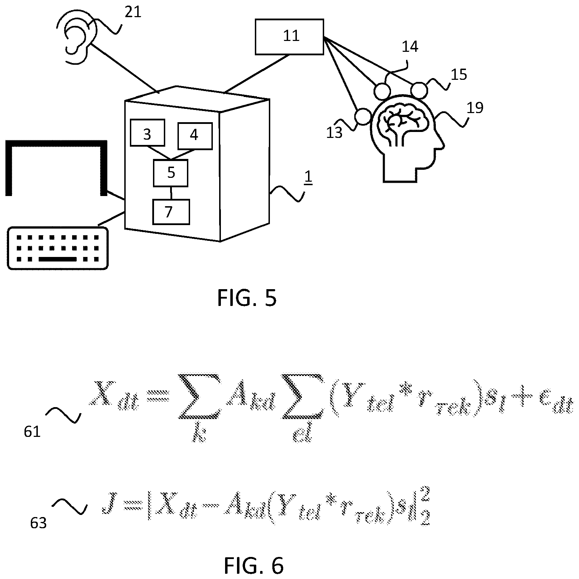

Fig. 5 shows a system for determining a person's sensory capabilities. In the embodiment of Fig. 5, the system is a computer 1. The computer 1 comprises a receiver 3, a transmitter 4, a processor 5, and storage means 7. The processor 5 is configured to obtain one or more brain wave signals via receiver 3 and obtain stimulus data representing a plurality of sensory stimuli presented over time with a plurality of levels. The one more brain wave signals are measured on a person 19 by a plurality of electrophysiological sensors 13-15 and obtained from processing device 11, which is connected to the sensors 13-15. Each of the stimuli is associated in the stimulus data with a level of the plurality of levels. In the embodiment of Fig. 5, the computer 1 presents the sensory stimuli to the person 19 and the processor 5 is configured to retrieve the stimulus data from storage means 7.

The processor 5 is further configured to determine a mathematical model in which the one or more brain wave signals are equal to an expression which comprises a sum of each of a plurality of spatial patterns multiplied with a factor representing activity of a corresponding neural source. The factor comprises a convolution of the stimulus data with the plurality of levels and stimulus responses for each of the neural sources. The stimulus responses are weighted with a stimulus response amplitude weight per level of the plurality of levels.

The processor 5 is also configured to estimate the plurality of spatial patterns, the stimulus responses, and the stimulus response amplitude weights in the mathematical model and determine the person's sensory capabilities based on the stimulus response amplitude weights. In the example of Fig. 5, the computer 1 is connected to a display and a keyboard for interacting with a worker who is conducting the test. The processor 5 may be configured to configure a hearing aid 21 based on the person's sensory capabilities via transmitter 4.

In the embodiment of the computer 1 shown in Fig. 5, the computer 1 comprises one processor 5. In an alternative embodiment, the computer 1 comprises multiple processors. The processor 5 of the computer 1 may be a general-purpose processor, e.g., from Intel or AMD, or an application-specific processor. The processor 5 of the computer 1 may run a Windows, iOS, or Unix-based operating system for example. The storage means 7 may comprise one or more memory units. The storage means 7 may comprise one or more hard disks and/or solid-state memory, for example. The storage means 7 may be used to store an operating system, applications and application data, for example.

The receiver 3 and the transmitter 4 may use one or more wired and/or wireless communication technologies such as Ethernet and/or Wi-Fi (IEEE 802.11) to communicate with processing device 11 and/or hearing aid 21, for example. In an alternative embodiment, multiple receivers and/or multiple transmitters are used instead of a single receiver and a single transmitter. In the embodiment shown in Fig. 5, a separate receiver and a separate transmitter are used. In an alternative embodiment, the receiver 3 and the transmitter 4 are combined into a transceiver. The computer 1 may comprise other components typical for a computer such as a power connector. The invention may be implemented using a computer program running on one or more processors.

The stimulus data/sequences may not only distinguish between an event being present and no event being present (stimulus off) but also between different event types. For example, the stimulus sequences may distinguish between at least two of the following event types for each particular moment at which an event is present:

1) stimulus-onset; the moment the stimulus goes from 'off (dark) to 'on' (bright)
2) stimulus-on; the period during which the stimulus remains 'on'
3) stimulus-offset; the moment the stimulus goes from 'on' to 'off.

For auditory testing, it is typically not necessary to distinguish between different event types, but it is sufficient for the stimulus sequences to indicate whether the stimulus is on or off. For visual testing, it is beneficial to distinguish between stimulus-onset and stimulus off-set events. Although it is possible to additional distinguish stimulus-on events, the brain responds much more strongly, and more specifically when stimuli change. Hence, responses to events of other event types may be treated as unmodelled background noise.

It is assumed that for each particular moment in the stimulus sequence, the shape of the response is the same (compared to responses to other stimuli, relative to the start of the stimulus), with only amplitude varying per moment in the stimulus sequence. Furthermore, the brain is expected to respond differently to two different types of events in the stimulus sequence. Thus, the stimulus sequence represents the sensory stimuli presented over time for each of the levels and for each of the selected event types, the stimulus responses are determined for each of the neural sources and each of the selected event types, and the stimulus response amplitude weights are independent of the event types.

The mathematical model of Fig. 3 is represented by a mathematical equation 61 in Fig. 6. The Eq. 61 on Fig. 6 has r, s, and y variables rearranged with respect to Fig. 3 for clarity. The rearrangement follows from the linearity property of convolution which includes associativity with scalar multiplication. Equation 61 shows that the measured brain wave signal X is decomposed into:

- k neural source dependent spatial patterns; these give an indication of the spatial location of the underlying neural source;
- e*k neural source and event-dependent stimulus (impulse) responses; these show how neural source k responds over time to an isolated stimulus event of type e; and
- a level-dependent weighting, $s_l$, which shows how all the responses are scaled with respect to different stimuli levels.

In equation 61 of Fig. 3, the measured brain wave signal X is equal to an expression which comprises a sum of each of the k spatial patterns multiplied with a factor representing activity of a corresponding neural source. The factor comprises a convolution of the stimulus data Y with the plurality of levels and stimulus responses r for each of the neural sources. The stimulus responses r are weighted with a stimulus response amplitude weight s per level (convolution is an associative operation). In equation 61 of Fig. 3, the above-mentioned expression further comprises a factor $\varepsilon$ representing unmodelled signal and noise.

Specifically,

- $X_{dt}$ is the measured data for d channels and t timepoints; This is a matrix comprising real numbers, normally representing processed measured data, e.g. processed EEG data.

- $A_{kd}$ is the set of spatial-patterns, one for each of k modelled sources; This is a matrix representing the spatial distribution of the modeled sources, k, and the channels, *d,* of the measured data. This matrix comprises real numbers.

- $Y_{tel}$ is the stimulus sequence for t time-points. This is a 0-1 indicator matrix where at each time point a stimulus is described as being of a particular 'type' e with a given 'level' l by placing a 1 at the location t,e,l;

- $r_{\tau ek}$ is the isolated stimulus response for events of type e for each of the modelled k sources. This response lasts for tau ($\tau$) time-points. $r_{\tau ek}$ is a tensor of order 3 (or a 3 dimensional array) with the dimension tau ($\tau$) representing the duration of the response potential, dimension e representing the type of stimuli, and dimension k representing the modelled sources. This tensor comprises real numbers. The stimulus responses may be stimulus response potentials (evoked response potentials), e.g. for EEG data, or stimulus response fields (evoked response fields), e.g. for MEG data ;

- * represents simple linear convolution of the event responses (of duration tau time-points) over the whole stimulus sequence duration of t samples;

- $s_l$ is the stimulus response amplitude weighting for each stimulus 'level' l (one weight per level); and

- $\varepsilon_{dt}$ represents un-modelled signal and noise.

Typically, each of the levels represents a stimulus feature for which the response amplitude is expected to vary. Each of the levels may represent an audio intensity or a position in the user's visual field, for example. For auditory testing, the levels may be determined for a plurality of audio intensities and a plurality of tones, wherein each of the levels represent a different combination of audio intensity and tone. In this case, an own unique pseudo-random noise sequence may be created for each of the tones. Each of these pseudo-random noise sequences specifies which audio intensity of the tone is to be played at a particular instant in time and may for example comprise a multi-level gold-code. Multi-level gold-codes have been described, for example, in "Five Shades of Grey: Exploring Quintary m-Sequences for More User-Friendly c-VEP-Based BCIs" by Felix W. Gembler et al., Computational Intelligence and Neuroscience, vol. 2020, Article ID 7985010, 11 pages, 2020.

These pseudo-random noise sequences are similar to those described in US 10,314,508 B2 but used for determining a person's sensory capabilities instead of for communicating with the person using a brain-computer interface (BCI). These pseudo-random noise sequences can be presented to the person simultaneously and thereby reduce testing time. In more detail, these stimuli may have the following key properties:

   1. Each tone has its own unique pseudo-random sequence, where this sequence specifies which audio intensity of that tone is to be played at a particular instant in time. This pseudo-random noise sequence is designed as a multi-level gold-code, such that in addition, the responses to different audio intensities are maximally uncorrelated from each other, and from themselves at different points in time.

   2. All tones are presented at the same time, but with unique multi-level gold codes for each tone. Codes used for different tones are designed such that the responses to different tones are maximally uncorrelated from each other, and from themselves at different points in time. However, the stimuli do not need to be limited to stimulating a single sensory pathway such as vision or hearing. Any combination of sensory pathways may be stimulated simultaneously where each sensory pathway is stimulated by a pseudo-random sequence. As described above, these pseudo-random sequences may be designed as multi-level gold-codes, such that in addition, the responses to different sensory stimuli features and levels are maximally uncorrelated from each other, and from themselves at different points in time.

Instead of a multi-level gold code, any other stimulus-sequence may be used. However, to maximize performance in terms of minimal data-gathering time a pseudo-random sequence that is maximally uncorrelated between tones and intensity levels should be used.

The advantages of the above design are:

- The complete brain response to an individual tone-audio intensity combination can be estimated directly from the measured data. This brain response is potentially a rich source of additional information on the users hearing capabilities. For example, in addition to the amplitude, the latency or location of the response may provide information about when and how the tone is being processed. Further, this brain response is potentially useful as a diagnostic tool for neurological disorders outside hearing, such as Alzheimer's.

- All tones are presented simultaneously, potentially allowing to stop a test earlier if the response is clear. All combinations of audio intensity and tone may even be presented simultaneously. Alternatively, when coupled with an active learning system, initial results from early in the test can be used to rapidly focus in on the transition region of the psycho-acoustic response curve, thereby giving a more accurate response curve estimate in less time.
- Extension to (adaptively) add more audio intensities or tones is straightforward by simply using more codes with more audio intensities. This offers the potential to obtain more detailed response curve estimates than simply 4-tones + 8-audio intensities.

Whilst uncorrelated pseudo-random codes are preferred for the above reasons, other stimulus-sequence may be used to maximize testing effectiveness. Developing the optimal stimulus sequence may require a trade-off between sequences with statistical properties which maximize the EP amplitude and user comfort (such as periodicity or rhythmicity) and sequences which maximize performance in terms of data-gathering time (which should be maximally uncorrelated between tones and intensity levels).

In equation 61 of Fig. 6, the stimulus sequence and stimulus response differ per event. Either one event type or a plurality of event types may be modelled. In a simple case, there is only one event type. For example, in a very simple visual/auditory 'can you see/hear' test, there might be a binary: stimulus / no-stimulus model, which basically means a single event type means 'stimulus-present'. In a more advanced case, multiple event types are distinguished. Certain stimulus parameters may be represented by an event type in addition to or instead of as a level. The decision on the number of event types vs. levels is a design choice for the modeler. The model fitting works for any number of events. The following are examples of choices for events and levels:

- Auditory testing: event= stimulus (i.e., one event type), levels = tone * audio intensity (i.e., unique combinations of tone and audio intensity). The response amplitude normally varies as the audio moves outside the users hearing range (extreme high/low frequencies).
- Visual testing - 'full-field' testing of visual acuity: event= stimulus-onset + stimulus-offset, levels=grid-spacing. The response amplitude normally varies when the grid-spacing goes below the user's visual resolution.
- Visual testing - 'multi-focal' testing of sensitivity at multiple positions: event= stimulus-onset + stimulus-offset, levels=position in the visual field * grid-spacing. The response amplitude normally also varies when the grid-spacing goes below the user's visual resolution and as the visual stimulus moves outside the user's range of vision (extreme periphery), or inside a visual deficit.

- Visual testing - 'face emotion processing' testing psychological disorders cognitive development : event = stimulus-onset, levels = neutral faces, fearful faces, happy faces. The response amplitude varies with neutral and fearful faces. This test may be used to screen for autism spectrum disorder (ASD), for example.
- Visual testing - 'face blindness' testing neurological disorders (such as prosopagnosia)//cognitive development event = stimulus-onset, levels = gradual deformation of the face. The response amplitude varies with gradual deformation of the face. This face does not have to be a familiar face.

The levels are used to model if the response amplitude varies with the stimulus parameter. Hence a parameter which is expected to vary in amplitude down to 0 is normally modelled with a level. As mentioned above, level and event are not exclusive, e.g., events may be partitioned over levels pretty much arbitrarily. For example, if the modeler thinks that there is a unique response shape for 500hz tones, vs 1000hz tones, he could use different events for 500hz and 1000hz with unique levels for each combination of audio intensity and tone. This would make the arrays a bit bigger in the mathematical model and slightly increase the number of parameters to estimate, but not by a large amount, particularly compared to the full unconstrained model.

Auditory testing and visual testing may be performed at the same time, i.e. auditory stimuli may be presented simultaneously with visual stimuli. In this case, there may be an event at the visual stimulus onset and an event at the auditory stimulus offset, for example. The auditory levels may be different combinations of tone and audio intensity, for example. The visual levels may be different grid spacings or different combinations of location in the user's visual field and grid spacing, for example.

**Model Estimation**

There are many possible techniques to estimate the parameters in this model given a dataset consisting of $X_{dt}$ and $Y_{tel}$. In the following paragraphs, two approaches are described:

  1. Minimum Least Squares Estimation (MLSE)
  2. Iterative Canonical Correlation Analysis (iCCA) Estimation

1. Minimum Least Squares Estimation

In this first approach, to fit the mathematical model, first, the least squares objective function 63 shown in Fig. 6 is used. For ease of writing, sub-script only Einstein notation has been adopted. Briefly, in this convention, repeated subscripts which do not appear on the other side of an equals sign are assumed to be summed over. The $|....|^2\_2$ is the notation for the 2-norm, i.e. quadratic norm, of what is between the bars $\|$. This objective function 63 can be directly minimized to fit the model parameters, e.g., by using an optimization algorithm such as (stochastic) gradient descent.

This objective function 63 is highly non-linear in the parameters ($r_{\tau ek}$, $s_l$ and $A_{kd}$), and suffers from degeneracies which can cause convergence issues. However, this model is linear in each parameter individually; if the other parameters are fixed, the result is a simple linear least squares in the remaining parameters. Thus the Alternating Least Squares (ALS) technique should be an effective algorithm. This objective function 63 is similar to the one used to find Parafac/CANDECOMP tensor decompositions, for which ALS is commonly used.

2. Iterative CCA Estimation

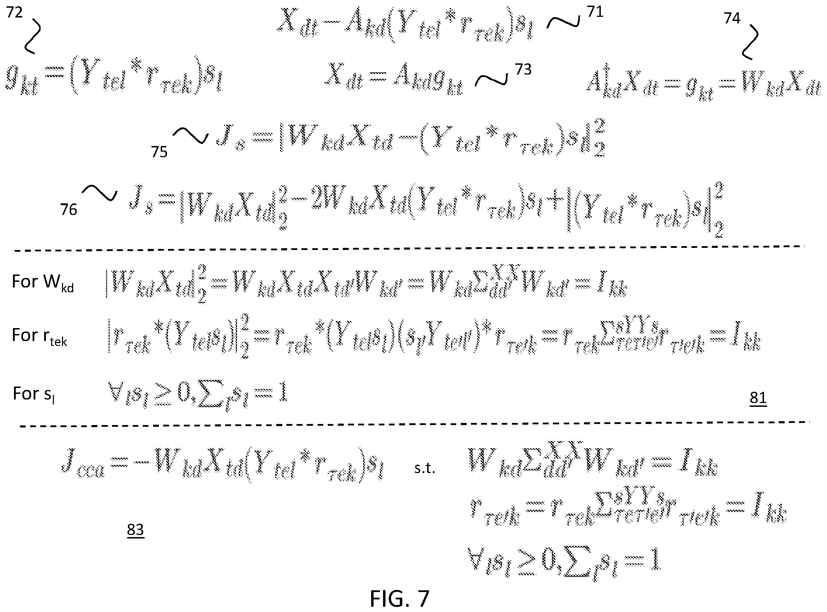

In the MLSE approach, the objective is to minimize the unmodelled residual 71 shown in Fig. 7. Implicit in this object are the following assumptions:

1. That the un modelled residual follows a (roughly) gaussian distribution; and
2. That the model captures a large fraction of the variance in X, or equivalently that the residual is a small fraction of X.

For many problems, these assumptions are reasonable, and the MLSE approach is appropriate. However, they are not reasonable for electrophysiological data, which has the following properties:

- Extreme outliers: due to the measurement process, electrophysiological data is prone to extremely large outliers due to measurement artifacts, such as subject movements, line-noise and un-modelled muscular activity. Thus, electrophysiological data commonly have a highly non-gaussian noise distribution
- Low modeling power: the measured electrophysiological signal contains a lot of information, only a very small fraction of which is related to the stimulus response that is of interest. Further, much of this uninteresting information, such as line-noise, muscle activity, or the activity of stimulus-independent brain processes, has a much higher amplitude than the stimulus response that needs to be extracted. Thus, modeling the signal of interest may only capture a small fraction of the variance in X.

Both of these problems can be avoided (to some extent) by minimizing the least squares loss in the source activity space (g) rather than in the measurement space (X), as this both suppresses or removes the non-gaussian artifacts and only computes the residual in the sub-space where the signal of interest lies. Two equations can be derived for the estimated source activity g.

1. A forward model, which estimates the source activity based on stimulus activity. This is shown as equation 72 in Fig. 7.
2. A backward model, which estimates the source activity based directly from the measured data X. This is shown as equation 73 in Fig. 7. From equation 73, equation 74 of Fig. 7 can be derived (assuming $A_{kd}$ has a defined pseudo-inverse).

Combining these definitions, the source-space least squares objective function 75, shown in Fig. 7, can be defined. As this objective function only considers the model quality in the estimated source sub-space, it is robust to the extreme artifact issues of the data-space least squares objective. However, it does suffer from a new problem of solution degeneracy, where a minimal solution can be found by simply setting $W_{kd}=0$, and either $s_l=0$ or $r_{\tau ek}=0$.

Fortunately, this issue can be avoided by constraining the magnitude of $W_{kd}$. For example, the degenerate solutions may simply be excluded from consideration by constraining the solutions to not allow any component to have an all zero-solution. This can be implemented by simply requiring that each component has a length equal to 1. This constraint is not needed in a simple least squares estimation, as in that case, the degenerate solutions are not also the optimal solutions as, an all zero solution is not a good fit, so has a high least-squares cost. Therefore, a good optimizer will ignore this and find a better solution.

Js of Equation 75 can be solved in many different ways, such as gradient descent, but as for $J_{ls}$, it is highly non-linear in its parameters and multi-modal with many solution degeneracies. Fortunately, many of these issues can be avoided by re-expressing the objective as an iterative Canonical Correlation Analysis problem. In equation 76 of Fig. 7, first, the brackets have been multiplied out and terms have been collected.

As mentioned earlier, the objective function suffers from a degeneracy if any (component of) of Wkd, $r_{\tau ek}$ or sl becomes all zero. To exclude this issue, norm constraints can be added on these terms. By choosing the right constraints, the first and last terms of Js can be forced to be constant and hence ignored. Specifically, constraints 81 of Fig. 7 are introduced for Wkd, for $r_{\tau ek}$, and for sl. Applying these constraints to Js, Jcca is obtained, as shown in Equation 83 of Fig. 7.

Fortunately, *Jcca* is the objective minimized given a fixed sl by the Canonical Correlation Algorithm (CCA), hence the name, for which there exist fast and efficient solutions based on matrix decomposition techniques. The notation of this constrained optimization problem, *Jcca*, in traditional CCA terminology could be written as (subject to the constraints mentioned above):

$$\underset{W_{kd}, r_{tek}}{\operatorname{argmax}}\Big(corr\left(W_{kd}\,X_{dt},\,(Y_{tel}\,*\,r_{\tau ek})s_l\right)\Big)$$

In R, function corr(x1,x2) calculates the correlation between two points x1 and x2 in the parameter space. Argmax is an operation that finds the argument that gives the maximum value from a target function. Importantly, these solutions do not suffer from the solution degeneracies mentioned above.

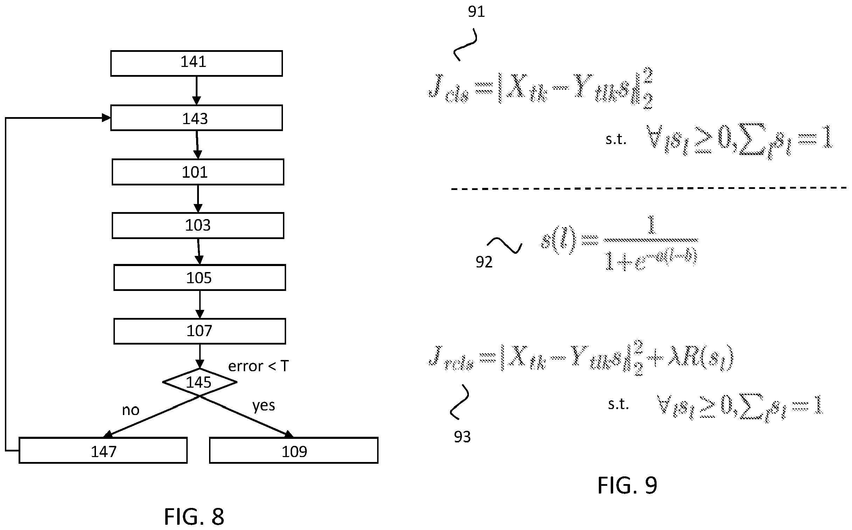

Finding the optimal value of sl given a fixed Wkd, rτek is a simple constrained least squares problem, Jcls, which is shown in equation 91 of Fig. 9. Again, many algorithms exist to solve this constrained optimization problem, such as projected gradient descent and Iterative Reweighted Least Squares. Combining these observations, leads to the following iterative CCA algorithm for estimating the model parameters:

> 1 While Js is still decreasing:
>
> > 2 Fix $s_l$ and find the optimal solution of $J_{cca}$ with respect to $W_{kd}\,r_{\tau ek}$
> > 3 Fix $W_{kd}\,r_{\tau ek}$ and find the optimal solution of $J_{cls}$ with respect to $s_l$

In order to determine the amount of data collection time needed, empirical experiments may be performed to work out how much data will be needed to reach the performance goals of a particular application. The model fitting may also be able to directly give an estimate of its own fit validity, which can then be used to adaptively decide if more data collection is needed. The model's estimate of its own fit quality can be computed as, but is not limited to, a simple cross validation-based measure of explained variance in the source subspace. Specifically, this can be computed in the following way:

> 1 For a number of folds, N, split the data into training and validation subsets;
> 2 Using the training subset, fit the model to estimate $W_{kd}\,r_{\tau ek}$ and $s_l$;
> 3 Apply this model to the validation subset to compute gx and gy;
> 4 The explained variance in the model sub-space is then corr(gx, gy); and
> 5 Average explained variance over folds to get a whole-dataset goodness-of-fit measure.

The goodness-of-fit measure computed in this way can be used to decide if more data gathering is needed; either by requiring that a minimum threshold goodness-of-fit level is reached or that the rate of improvement of goodness-of-fit is near zero. Additionally or alternatively, this measure of goodness-of-fit may be used to compare a plurality of models which vary on the encoding between the stimuli and the levels, or the constraints added to the estimation of $s_l$ (see section "Adding additional constraints on $s_l$" below). The model that explains the empirical data and the stimuli in the most correct fashion may then be selected from this plurality of models. For example, the model with the highest goodness-of-fit or a goodness-of-fit exceeding a threshold may be selected. The best mathematical model may be selected, for example, before a test is performed with a patient, e.g. using previous measurement data relating to test subjects, or after a test is performed with the patient, e.g. using current measurement data relating to the patient.

As an alternative to computing a goodness-of-fit measure, model parameter confidence-intervals may be computed with a re-sampling approach to decide if sufficient data has been obtained, as outlined below:

> 1 For a number of resamples, say 10, sample (with replacement) N samples from the data set;
> 2 Fit the model on this sample to estimate $W_{kd}\,r_{\tau ek}$ and $s_l$; and
> 3 Use the set of model parameter estimates to compute a modal set of parameters and their spread over resamples.

With this approach a 'model-stability' score can be computed based on the parameter spread over resamplings to decide if more data gathering is needed; either by requiring a minimum threshold stability or requiring that the rate of improvement of model stability is sufficiently near zero. This approach is particularly appealing when particular parameters are important for later decision making, for example if the sl is used to determine visual acuity then it is typically important to require that its estimated value has a sufficiently smaller estimation error. This approach allows for statistical inference on the differences between the distributions of weights $s_l$ of each level after resampling.

### Adding additional constraints on si

In many practical cases, it is beneficial to impose additional constraints on the stimulus dependent amplitude response which is modelled with the sl (weight) parameters. For example, in a stimulus detection test, sl may be required to follow a particular type of psychometric function, with a sigmoid like shape, or to be sufficiently smooth, such that the response level is similar for stimuli with similar properties. Such additional constraints may be simply achieved by either;

> 1. Providing a parametric model for sl and optimizing directly over the parameters. For example, when using parametric model 92 of Fig. 9, where a,b are the slope and threshold of the logistic psychometric curve, the loss Jcls can be directly optimized with respect to these parameters as before.
> 2. Adding an additional regularization term , R(sl), to penalize sl configurations which are not preferred. This results in the modified optimization problem 93, shown in Fig. 9. Again, this sub-problem can be solved by any standard constrained regularized least squares optimizer.

The above-mentioned additional constraints on sl are optional and represent additional assumptions on the form of the brain response. Thus, adding them is a trade-off between:

> a) reducing the data-collection time if the assumptions are correct
> b) increasing the data-collection time (possibly to infinity) if the assumptions are incorrect.

These additional constraints and the associated trade-off is equally applicable to the MLSE and CCA estimators. If a parametric model is used for sl which further includes error estimates for the estimated amplitudes for the different stimulus parameters, e.g., a gaussian process model, it may be possible to significantly reduce the total testing time required by using Active Learning techniques to adaptively present stimuli to the user which maximally reduce the estimated error.

In particular for testing problems with known structure, such as the relative smoothness of the localized amplitude response in vision or auditory testing, by focusing testing examples near the detection threshold this technique can further massively reduce the total testing time. In other words, in combination with an appropriate active learning system, stimuli can be selected adaptively in such a way that the model estimate is improved as rapidly as possible, further reducing the amount of data required.

Fig. 8 shows an embodiment of the computer-implemented method of determining a person's sensory capabilities in which active learning is used. A step 141 comprises determining a plurality of initial levels, e.g. default levels. A step 143 comprises presenting a plurality of sensory stimuli with the plurality of levels determined in e.g., step 141.

Step 101 comprises obtaining one or more brain wave signals. The one or more brain wave signals are measured on the person by a plurality of electrophysiological sensors. Step 103 comprises obtaining stimulus data representing a plurality of sensory stimuli presented over time with a plurality of levels. Each of the stimuli are associated in the stimulus data with a level of the plurality of levels.

Sep 105 comprises determining a mathematical model in which the one or more brain wave signals are equal to an expression which comprises a sum of each of a plurality of spatial patterns multiplied with a factor representing activity of a corresponding neural source. The factor comprises a convolution of the stimulus data with the plurality of levels and stimulus responses for each of the neural sources. The stimulus responses are weighted with a stimulus response amplitude weight per level of the plurality of levels.

In the embodiment of Fig. 8, the stimulus response amplitude weights are constrained, as previously described. The stimulus response amplitude weights may be constrained to follow a smooth function and/or a psychometric function with a sigmoid-like shape, for example. In the embodiment of Fig. 8, a parametric model is used for sl which further includes error estimates for the estimated amplitudes for the different stimulus parameters. In an alternative embodiment, the stimulus response amplitude weights are not constrained.

Step 107 comprises estimating the plurality of spatial patterns, the stimulus responses, and the stimulus response amplitude weights in the mathematical model. A step 145 comprises determining whether the above-mentioned estimated error stays below a threshold T. If so, step 109 is performed. If not, a step 147 is performed.

Step 147 comprises determining a further plurality of levels. These levels are selected using active learning techniques. Such techniques, include, but are not limited to, selecting the levels which currently have the largest estimation error when using an Uncertainty Sampling strategy, or those with largest estimated variance when using a Variance Maximization strategy, or those with the largest effect on the margin when using a Margin Sampling strategy or for which the current model has the least confidence when using a Least Confidence strategy (see e.g. https://www.datacamp.com/community/tutorials/active-learning).

Step 143 is repeated after step 147, and the method proceeds with the next iteration in the manner shown in Fig. 8. In the next iteration of step 143, a further plurality sensory stimuli is presented with the further plurality of levels. In the next iterations of step 103, 105, and 107, only the latest stimulus data and brain wave signals may be used or also stimulus data and brain wave signals of previous iterations, e.g., of all previous iterations, may be used.

Step 109 comprises determining the person's sensory capabilities based on at least the stimulus response amplitude weights determined in the last iteration of step 107. Optionally, the person's sensory capabilities are further determined based on the stimulus responses and/or the spatial patterns.

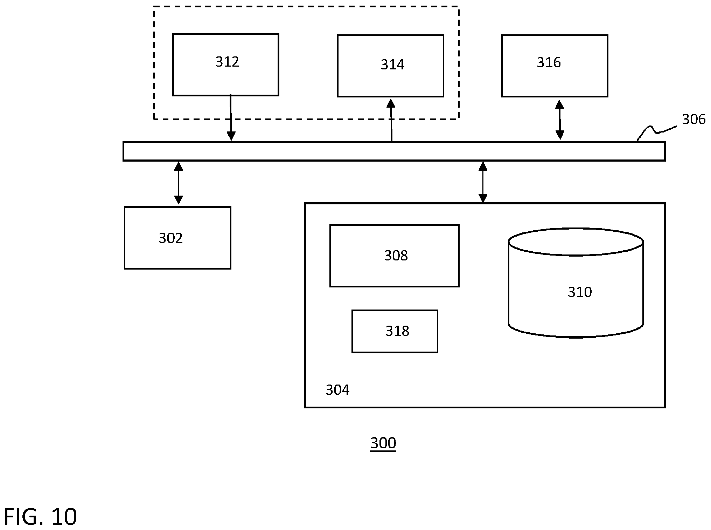

Fig. 10 depicts a block diagram illustrating an exemplary data processing system that may perform the method as described with reference to Figs. 1, 2, and 8.

As shown in Fig. 10, the data processing system 300 may include at least one processor 302 coupled to memory elements 304 through a system bus 306. As such, the data processing system may store program code within memory elements 304. Further, the processor 302 may execute the program code accessed from the memory elements 304 via a system bus 306. In one aspect, the data processing system may be implemented as a computer that is suitable for storing and/or executing program code. It should be appreciated, however, that the data processing system 300 may be implemented in the form of any system including a processor and a memory that is capable of performing the functions described within this specification.

The memory elements 304 may include one or more physical memory devices such as, for example, local memory 308 and one or more bulk storage devices 310. The local memory may refer to random access memory or other non-persistent memory device(s) generally used during actual execution of the program code. A bulk storage device may be implemented as a hard drive or other persistent data storage device. The processing system 300 may also include one or more cache memories (not shown) that provide temporary storage of at least some program code in order to reduce the number of times program code must be retrieved from the bulk storage device 310 during execution.

Input/output (I/O) devices depicted as an input device 312 and an output device 314 optionally can be coupled to the data processing system. Examples of input devices may include, but are not limited to, a keyboard, a pointing device such as a mouse, or the like. Examples of output devices may include, but are not limited to, a monitor or a display, speakers, or the like. Input and/or output devices may be coupled to the data processing system either directly or through intervening I/O controllers.

In an embodiment, the input and the output devices may be implemented as a combined input/output device (illustrated in Fig. 10 with a dashed line surrounding the input device 312 and the output device 314). An example of such a combined device is a touch sensitive display, also sometimes referred to as a "touch screen display" or simply "touch screen". In such an embodiment, input to the device may be provided by a movement of a physical object, such as e.g. a stylus or a finger of a user, on or near the touch screen display.

A network adapter 316 may also be coupled to the data processing system to enable it to become coupled to other systems, computer systems, remote network devices, and/or remote storage devices through intervening private or public networks. The network adapter may comprise a data receiver for receiving data that is transmitted by said systems, devices and/or networks to the data processing system 300, and a data transmitter for transmitting data from the data processing system 300 to said systems, devices and/or networks. Modems, cable modems, and Ethernet cards are examples of different types of network adapter that may be used with the data processing system 300.

As pictured in Fig. 10, the memory elements 304 may store an application 318. In various embodiments, the application 318 may be stored in the local memory 308, he one or more bulk storage devices 310, or separate from the local memory and the bulk storage devices. It should be appreciated that the data processing system 300 may further execute an operating system (not shown in Fig. 10) that can facilitate execution of the application 318. The application 318, being implemented in the form of executable program code, can be executed by the data processing system 300, e.g., by the processor 302. Responsive to executing the application, the data processing system 300 may be configured to perform one or more operations or method steps described herein.

Various embodiments of the disclosure may be implemented as a program product for use with a computer system, where the program(s) of the program product define functions of the embodiments (including the methods described herein). In one embodiment, the program(s) can be contained on a variety of non-transitory computer-readable storage media, where, as used herein, the expression "non-transitory computer readable storage media" comprises all computer-readable media, with the sole exception being a transitory, propagating signal. In another embodiment, the program(s) can be contained on a variety of transitory computer-readable storage media. Illustrative computer-readable storage media include, but are not limited to: (i) non-writable storage media (e.g., read-only memory devices within a computer such as CD-ROM disks readable by a CD-ROM drive, ROM chips or any type of solid-state non-volatile semiconductor memory) on which information is permanently stored; and (ii) writable storage media (e.g., flash memory, floppy disks within a diskette drive or hard-disk drive or any type of solid-state random-access semiconductor memory) on which alterable information is stored. The computer program may be run on the processor 302 described herein.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of embodiments of the present invention has been presented for purposes of illustration, but is not intended to be exhaustive or limited to the implementations in the form disclosed. Many modifications and variations will be apparent to those of ordinary skill in the art without departing from the scope of the present invention. The embodiments were chosen and described in order to best explain the principles and some practical applications of the present invention, and to enable others of ordinary skill in the art to understand the present invention for various embodiments with various modifications as are suited to the particular use contemplated.

The invention is defined with a system according to claim 1.

**Claims**

1.  A system (1) for determining a person's (19) sensory capabilities and/or a psychological and/or neurological state of the person, the system (1) comprising at least one processor (5) configured to:

    - obtain one or more brain wave signals, the one more brain wave signals being measured on the person (19) by a plurality of electrophysiological sensors (13-15),
    - obtain stimulus data representing a plurality of sensory stimuli presented over time with a plurality of levels, the stimulus data lasting for a plurality of time points, each of the stimuli being associated in the stimulus data with a level of the plurality of levels and lasting for a subset of the plurality of time points, **characterised in that** the at least one processor is further configured to:

    - determine a mathematical model in which the one or more brain wave signals are equal to an expression which comprises a sum of each of a plurality of spatial patterns multiplied with a factor representing activity of a corresponding underlying neural source, the factor comprising a convolution of the stimulus data and isolated stimulus responses for each of the neural sources, the isolated stimulus responses being weighted with a stimulus response amplitude weight per level of the plurality of levels, each of the spatial patterns being indicative of a spatial location of the corresponding underlying neural source,
    - estimate the plurality of spatial patterns, the stimulus responses, and the stimulus response amplitude weights in the mathematical model, and
    - determine the person's (19) sensory capabilities and/or the psychological and/or neurological state of the person based on the stimulus response amplitude weights.

2.  A system as claimed in claim 1, wherein the stimulus data indicate the periods during which the stimuli are on.

3.  A system as claimed in claim 1 or 2, wherein a plurality of events of different event types are distinguished in each of the stimuli, the stimulus data represents the sensory stimuli presented over time for each of the levels and for each of the event types, the isolated stimulus responses are determined for each of the neural sources and each of the event types, and the stimulus response amplitude weights are independent of the event types.

4.  A system as claimed in claim 3, wherein one of the event types represents an onset moment of the stimuli and/or one of the event types represents an offset moment of the stimuli.

5.  A system as claimed in any one of the preceding claims, wherein the expression further comprises a factor representing unmodelled signal and noise.

6.  A system as claimed in any one of the preceding claims, wherein each of the levels represents an audio intensity, a position in the user's visual field, a degree of contrast in luminance and/or color, a visual spatial resolution, a degree of familiarity with a complex visual stimulus, a degree of deformation of a complex auditory stimulus, or a degree of deformation of a complex visual stimulus.

7.  A system as claimed in claim 6, wherein the levels have been determined for a plurality of audio intensities and a plurality of tones, each of the levels representing a different combination of audio intensity and tone.

8.  A system as claimed in claim 6, wherein the levels have been determined for a plurality of degrees of contrast in luminance and/or color and a plurality of positions in the user's visual field, each of the levels representing a different combination of degree of contrast in luminance and/or color and location in the user's visual field.

9.  A system as claimed in claim 6, wherein the levels have been determined for a plurality of degrees of color contrast and a plurality of degrees of luminance contrast at a single location in the user's visual field, each of the levels representing a different combination of degree of color contrast and degree of luminance contrast.

10. A system as claimed in claim 6, wherein the levels have been determined for a plurality of degrees of contrast in color and/or luminance and a plurality of visual spatial resolutions, each of the levels representing a different combination of degree of contrast in color and/or luminance and visual spatial resolution.

11. A system as claimed in claim 6, wherein the complex visual stimulus comprises an image of a face and/or the complex auditory stimulus comprises a phonetic sound.

**12.** A system as claimed in any one of the preceding claims, wherein the at least one processor is configured to create a unique pseudo-random sequence for each of a plurality of sensory stimulus features, each of the pseudo-random sequences specifying which of the plurality of levels of the corresponding sensory stimulus feature is to be presented at a particular instant in time, and present the plurality of sensory stimulus features at the plurality of levels as specified by the pseudo-random sequences.

**13.** A system as claimed in claim 12, wherein the plurality of sensory stimulus features comprises a plurality of tones, the plurality of levels comprises a plurality of audio intensities, and each of the pseudo-random sequences specifies which of the plurality of audio intensities of the corresponding tone is to be played at a particular instant in time.

**14.** A system as claimed in claim 12 or 13, wherein the plurality of sensory stimulus features comprises a plurality of visual stimulus features and each of the pseudo-random sequences specifies at which particular location of the user's visual field and at which particular instant in time the corresponding visual stimulus feature is to be presented.

**15.** A system as claimed in any one of the preceding claims, wherein the at least one processor is configured to measure (121) the one or more brain wave signals and/or configure (125) a hearing aid and/or a sight correction aid based on the person's sensory processing capabilities.

**16.** A system as claimed in any one of the preceding claims, wherein the at least one processor is configured to determine (109) the person's sensory capabilities by determining (123) an audiometric threshold, a contrast sensitivity threshold, and/or a visual acuity threshold.

**17.** A system as claimed in any one of the preceding claims, wherein the stimulus response amplitude weights are constrained.

**18.** A system as claimed in claim 17, wherein the stimulus response amplitude weights are constrained to follow a smooth function and/or a psychometric function with a sigmoid-like shape.

**19.** A system as claimed in any one of the preceding claims, wherein the at least one processor is configured use re-sampling to determine a model parameter confidence interval of the stimulus response amplitude weights to statistically infer differences between the distributions of the stimulus response amplitude weights after re-sampling.

**20.** A system as claimed in any one of the preceding claims, wherein the at least one processor is configured to determine a measure of goodness-of-fit for each of a plurality of mathematical models, the plurality of mathematical models differing in used sensory stimuli and/or in used constraints on parameters of the mathematical model, and select one of the plurality of models based on the determined measures of goodness-of-fit of the model.

**Patentansprüche**

**1.** System (1) zum Bestimmen sensorischer Fähigkeiten einer Person (19) und/oder eines psychologischen und/oder neurologischen Zustandes der Person, wobei das System (1) zumindest einen Prozessor (5) aufweist, der dazu konfiguriert ist:

- ein oder mehrere Gehirnwellensignale zu erhalten, wobei das eine oder die mehreren Gehirnwellensignale an der Person (19) durch eine Mehrzahl von elektrophysiologischen Sensoren (13-15) gemessen werden,
- Reizdaten zu erhalten, die eine Mehrzahl von sensorischen Reizen darstellen, die über die Zeit mit einer Mehrzahl von Pegeln präsentiert werden, wobei die Reizdaten für eine Mehrzahl von Zeitpunkten andauern, wobei jeder der Reize in den Reizdaten mit einem Pegel der Mehrzahl von Pegeln verknüpft ist und für eine Teilmenge der Mehrzahl von Zeitpunkten andauert, **dadurch gekennzeichnet, dass** der zumindest eine Prozessor weiterhin dazu konfiguriert ist:

- ein mathematisches Modell zu bestimmen, in dem das eine oder die mehreren Gehirnwellensignale gleich einem Ausdruck sind, der eine Summe von jedem einer Mehrzahl von räumlichen Mustern multipliziert mit einem Faktor aufweist, der eine Aktivität einer entsprechenden zugrunde liegenden neuronalen Quelle darstellt, wobei der Faktor eine Faltung der Reizdaten und isolierter Reizantworten für jede der neuronalen Quellen aufweist, wobei die isolierten Reizantworten mit einem Reizantwortamplitudengewicht pro Pegel der Mehrzahl von Pegeln gewichtet sind, wobei jedes der räumlichen Muster einen räumlichen Ort der entsprechenden zugrunde liegenden neuronalen Quelle anzeigt,
- die Mehrzahl von räumlichen Mustern, die Reizantworten und die Reizantwortamplitudengewichte in dem mathematischen Modell zu schätzen, und
- die sensorischen Fähigkeiten der Person (19) und/oder den psychologischen und/oder neurologischen Zustand der Person basierend auf den Reizantwortamplitudengewichten zu bestimmen.

2. System nach Anspruch 1, wobei die Reizdaten die Zeiträume angeben, während der die Reize vorhanden sind.

3. System nach Anspruch 1 oder 2, wobei eine Mehrzahl von Ereignissen unterschiedlicher Ereignisarten in jedem der Reize unterschieden werden, die Reizdaten die sensorischen Reize darstellen, die über die Zeit für jeden der Pegel und für jede der Ereignisarten präsentiert werden, die isolierten Reizantworten für jede der neuronalen Quellen und jede der Ereignisarten bestimmt werden, und die Reizantwortamplitudengewichte unabhängig von den Ereignisarten sind.

4. System nach Anspruch 3, wobei eine der Ereignisarten einen Anfangszeitpunkt der Reize und/oder eine der Ereignisarten einen Endzeitpunkt der Reize darstellt.

5. System nach einem der vorhergehenden Ansprüche, wobei der Ausdruck weiterhin einen Faktor aufweist, der unmodelliertes Signal und Rauschen darstellt.

6. System nach einem der vorhergehenden Ansprüche, wobei jeder der Pegel eine Audiointensität, eine Position im Gesichtsfeld des Benutzers, einen Kontrastgrad in Luminanz und/oder Farbe, eine visuelle räumliche Auflösung, einen Vertrautheitsgrad mit einem komplexen visuellen Reiz, einen Deformationsgrad eines komplexen auditiven Reizes oder einen Deformationsgrad eines komplexen visuellen Reizes darstellt.

7. System nach Anspruch 6, wobei die Pegel für eine Mehrzahl von Audiointensitäten und eine Mehrzahl von Tönen bestimmt wurden, wobei jeder der Pegel eine unterschiedliche Kombination von Audiointensität und Ton darstellt.

8. System nach Anspruch 6, wobei die Pegel für eine Mehrzahl von Kontrastgraden in Luminanz und/oder Farbe und eine Mehrzahl von Positionen in dem Gesichtsfeld des Benutzers bestimmt wurden, wobei jeder der Pegel eine unterschiedliche Kombination von Kontrastgrad in Luminanz und/oder Farbe und Ort im Gesichtsfeld des Benutzers darstellt.

9. System nach Anspruch 6, wobei die Pegel für eine Mehrzahl von Farbkontrastgraden und einer Mehrzahl von Luminanzkontrastgraden an einem einzelnen Ort im Gesichtsfeld des Benutzers bestimmt wurden, wobei jeder der Pegel eine unterschiedliche Kombination an Farbkontrastgrad und Luminanzkontrastgrad darstellt.

10. System nach Anspruch 6, wobei die Pegel für eine Mehrzahl von Kontrastgraden in Farbe und/oder Luminanz und eine Mehrzahl von visuellen räumlichen Auflösungen bestimmt wurden, wobei jeder der Pegel eine unterschiedliche Kombination von Kontrastgrad in Farbe und/oder Luminanz und visueller räumlicher Auflösung darstellt.

11. System nach Anspruch 6, wobei der komplexe visuelle Reiz ein Bild eines Gesichtes aufweist, und/oder der komplexe auditive Reiz einen phonetischen Klang aufweist.

12. System nach einem der vorhergehenden Ansprüche, wobei der zumindest eine Prozessor dazu konfiguriert ist, eine eindeutige pseudozufällige Sequenz für jedes einer Mehrzahl von sensorischen Reizmerkmalen zu erzeugen, wobei jede der pseudozufälligen Sequenzen spezifiziert, welcher der Mehrzahl von Pegeln des entsprechenden sensorischen Reizmerkmals an einem bestimmten Zeitpunkt zu präsentieren ist, und die Mehrzahl von sensorischen Reizmerkmalen bei der Mehrzahl von Pegeln zu präsentieren, wie von den pseudozufälligen Sequenzen spezifiziert wird.

13. System nach Anspruch 12, wobei die Mehrzahl von sensorischen Reizmerkmalen eine Mehrzahl von Tönen aufweist, wobei die Mehrzahl von Pegeln eine Mehrzahl von Audiointensitäten aufweist, und wobei jede der pseudozufälligen Sequenzen spezifiziert, welche der Mehrzahl von Audiointensitäten des entsprechenden Tons zu einem bestimmten Zeitpunkt abzuspielen ist.

14. System nach Anspruch 12 oder 13, wobei die Mehrzahl von sensorischen Reizmerkmalen eine Mehrzahl von visuellen Reizmerkmalen aufweist und jede der pseudozufälligen Sequenzen spezifiziert, an welchem bestimmten Ort des Gesichtsfelds des Benutzers und zu welchem bestimmten Zeitpunkt das entsprechende visuelle Reizmerkmal zu präsentieren ist.

15. System nach einem der vorhergehenden Ansprüche, wobei der zumindest eine Prozessor dazu konfiguriert ist, das eine oder die mehreren Gehirnwellensignale zu messen (121) und/oder eine Hörhilfe und/oder eine Sehkorrekturhilfe basierend auf den sensorischen Verarbeitungsfähigkeiten der Person zu konfigurieren (125).

16. System nach einem der vorhergehenden Ansprüche, wobei der zumindest eine Prozessor dazu konfiguriert ist, die sensorischen Fähigkeiten der Person durch Bestimmen (123) eines audiometrischen Schwellwertes, eines Kontrastempfindlichkeitsschwellwertes und/oder eines Sehschärfenschwellwertes zu bestimmen (109).

17. System nach einem der vorhergehenden Ansprüche, wobei die Reizantwortamplitudengewichte eingeschränkt sind.

18. System nach Anspruch 17, wobei die Reizantwortamplitudengewichte eingeschränkt sind, um einer glatten Funktion und/oder einer psychometrischen Funktion mit einer Sigmoid-artigen Form zu folgen.

19. System nach einem der vorhergehenden Ansprüche, wobei der zumindest eine Prozessor dazu konfiguriert ist, ein erneutes Abtasten zu verwenden, um ein Modellparameterkonfidenzintervall der Reizantwortamplitudengewichte zu bestimmen, um Unterschiede zwischen den Verteilungen der Reizantwortamplitudengewichte nach erneutem Abtasten statistisch abzuleiten.

20. System nach einem der vorhergehenden Ansprüche, wobei der zumindest eine Prozessor dazu konfiguriert ist, ein Anpassungsgütemaß für jedes einer Mehrzahl von mathematischen Modellen zu bestimmen, wobei die Mehrzahl von mathematischen Modellen sich in den verwendeten sensorischen Reizen und/oder in den verwendeten Beschränkungen von Parametern des mathematischen Modells unterscheiden, und eines der Mehrzahl von Modellen basierend auf den bestimmten Anpassungsgütemaßen des Modells auszuwählen.

## Revendications

1. Système (1) de détermination des capacités sensorielles d'une personne (19) et/ou d'un état psychologique et/ou neurologique de la personne, le système (1) comprenant au moins un processeur (5) configuré pour :

   - obtenir un ou plusieurs signaux d'ondes cérébrales, les un ou plusieurs signaux d'ondes cérébrales étant mesurés sur la personne (19) par une pluralité de capteurs électrophysiologiques (13-15),
   - obtenir des données de stimuli représentant une pluralité de stimuli sensoriels présentés au fil du temps avec une pluralité de niveaux, les données de stimuli ayant une durée correspondant à une pluralité d'instants, chacun des stimuli étant associé dans les données de stimuli à un niveau de la pluralité de niveaux et ayant une durée correspondant à un sous-ensemble de la pluralité d'instants, **caractérisé en ce que** l'au moins un processeur est en outre configuré pour :

   - déterminer un modèle mathématique dans lequel les un ou plusieurs signaux d'ondes cérébrales sont égaux à une expression qui comprend une somme de chacune d'une pluralité de configurations spatiales multipliée par un facteur représentant l'activité d'une source neuronale sous-jacente correspondante, le facteur comprenant une convolution des données de stimuli et des réponses à un stimulus isolées pour chacune des sources neuronales, les réponses à un stimulus isolées étant pondérées avec une pondération d'amplitude de réponse à un stimulus pour chaque niveau de la pluralité de niveaux, chacune des configurations spatiales indiquant une position spatiale de la source neuronale sous-jacente correspondante,
   - estimer la pluralité de configurations spatiales, les réponses à un stimulus et les pondérations d'amplitude de réponse à un stimulus dans le modèle mathématique, et
   - déterminer les capacités sensorielles de la personne (19) et/ou l'état psychologique et/ou neurologique de la personne sur la base des pondérations d'amplitude de réponse à un stimulus.

2. Système selon la revendication 1, dans lequel les données de stimuli indiquent les périodes pendant lesquelles les stimuli sont activés.

3. Système selon la revendication 1 ou 2, dans lequel une pluralité d'événements de différents types d'événements sont distingués dans chacun des stimuli, les données de stimuli représentent les stimuli sensoriels présentés au fil du temps pour chacun des niveaux et pour chacun des types d'événements, les réponses à un stimulus isolées sont déterminées pour chacune des sources neuronales et chacun des types d'événements, et les pondérations d'amplitude de réponse à un stimulus sont indépendantes des types d'événements.

4. Système selon la revendication 3, dans lequel l'un des types d'événements représente un moment d'apparition des stimuli et/ou l'un des types d'événements représente un moment de disparition des stimuli.

5. Système selon l'une quelconque des revendications précédentes, dans lequel l'expression comprend en outre un facteur représentant un signal et un bruit non modélisés.

**6.** Système selon l'une quelconque des revendications précédentes, dans lequel chacun des niveaux représente une intensité audio, une position dans le champ de vision de l'utilisateur, un degré de contraste de luminance et/ou de couleur, une résolution spatiale visuelle, un degré de familiarité avec un stimulus visuel complexe, un degré de déformation d'un stimulus auditif complexe ou un degré de déformation d'un stimulus visuel complexe.

**7.** Système selon la revendication 6, dans lequel les niveaux ont été déterminés pour une pluralité d'intensités audio et une pluralité de tonalités, chacun des niveaux représentant une combinaison différente d'intensité audio et de tonalité.

**8.** Système selon la revendication 6, dans lequel les niveaux ont été déterminés pour une pluralité de degrés de contraste de luminance et/ou de couleur et une pluralité de positions dans le champ de vision de l'utilisateur, chacun des niveaux représentant une combinaison différente de degré de contraste de luminance et/ou de couleur et de position dans le champ de vision de l'utilisateur.

**9.** Système selon la revendication 6, dans lequel les niveaux ont été déterminés pour une pluralité de degrés de contraste de couleur et une pluralité de degrés de contraste de luminance à une seule position dans le champ de vision de l'utilisateur, chacun des niveaux représentant une combinaison différente de degré de contraste de couleur et de degré de contraste de luminance.

**10.** Système selon la revendication 6, dans lequel les niveaux ont été déterminés pour une pluralité de degrés de contraste de couleur et/ou de luminance et une pluralité de résolutions spatiales visuelles, chacun des niveaux représentant une combinaison différente de degré de contraste de couleur et/ou de luminance et de résolution spatiale visuelle.

**11.** Système selon la revendication 6, dans lequel le stimulus visuel complexe comprend une image d'un visage et/ou le stimulus auditif complexe comprend un son phonétique.

**12.** Système selon l'une quelconque des revendications précédentes, dans lequel l'au moins un processeur est configuré pour créer une séquence pseudo-aléatoire unique pour chacune d'une pluralité de caractéristiques de stimulus sensoriel, chacune des séquences pseudo-aléatoires spécifiant quel niveau parmi la pluralité de niveaux de la caractéristique de stimulus sensoriel correspondante doit être présenté à un instant particulier, et pour présenter la pluralité de caractéristiques de stimulus sensoriel à la pluralité de niveaux tels que spécifiés par les séquences pseudo-aléatoires.

**13.** Système selon la revendication 12, dans lequel la pluralité de caractéristiques de stimulus sensoriel comprend une pluralité de tonalités, la pluralité de niveaux comprend une pluralité d'intensités audio et chacune des séquences pseudo-aléatoires spécifie quelle intensité audio parmi la pluralité d'intensités audio de la tonalité correspondante doit être diffusée à un instant particulier.

**14.** Système selon la revendication 12 ou 13, dans lequel la pluralité de caractéristiques de stimulus sensoriel comprend une pluralité de caractéristiques de stimulus visuel et chacune des séquences pseudo-aléatoires spécifie à quelle position particulière du champ de vision de l'utilisateur et à quel instant particulier la caractéristique de stimulus visuel correspondante doit être présentée.

**15.** Système selon l'une quelconque des revendications précédentes, dans lequel l'au moins un processeur est configuré pour mesurer (121) les un ou plusieurs signaux d'ondes cérébrales et/ou pour configurer (125) une aide auditive et/ou un dispositif de correction de la vue sur la base des capacités d'intégration sensorielle de la personne.

**16.** Système selon l'une quelconque des revendications précédentes, dans lequel l'au moins un processeur est configuré pour déterminer (109) les capacités sensorielles de la personne en déterminant (123) un seuil audiométrique, un seuil de sensibilité différentielle et/ou un seuil d'acuité visuelle.

**17.** Système selon l'une quelconque des revendications précédentes, dans lequel les pondérations d'amplitude de réponse à un stimulus sont contraintes.

**18.** Système selon la revendication 17, dans lequel les pondérations d'amplitude de réponse à un stimulus sont contraintes de suivre une fonction de lissage et/ou une fonction psychométrique avec une forme de type sigmoïde.

**19.** Système selon l'une quelconque des revendications précédentes, dans lequel l'au moins un processeur est configuré pour utiliser un rééchantillonnage afin de déterminer un intervalle de confiance de paramètre de modèle des pondérations d'amplitude de réponse à un stimulus pour déduire statistiquement des différences entre les distributions des pondérations d'amplitude de réponse à un stimulus après le rééchantillonnage.

**20.** Système selon l'une quelconque des revendications précédentes, dans lequel l'au moins un processeur est configuré pour déterminer une mesure d'adéquation pour chacun d'une pluralité de modèles mathématiques, la pluralité de modèles mathématiques différant par les stimuli sensoriels utilisés et/ou les contraintes utilisées sur les paramètres du modèle mathématique, et pour sélectionner l'un de la pluralité de modèles sur la base des mesures d'adéquation déterminées du modèle.

EP 4 395 641 B1

```
┌─────────────────┐
│       101       │
└─────────────────┘
         │
         ▼
┌─────────────────┐
│       103       │
└─────────────────┘
         │
         ▼
┌─────────────────┐
│       105       │
└─────────────────┘
         │
         ▼
┌─────────────────┐
│       107       │
└─────────────────┘
         │
         ▼
┌─────────────────┐
│       109       │
└─────────────────┘
```

FIG. 1

```
┌───────────────────────────┐
│  ┌─────────────────┐       │
│  │      121        │  101  │
│  └─────────────────┘       │
└───────────────────────────┘
         │
         ▼
┌─────────────────┐
│       103       │
└─────────────────┘
         │
         ▼
┌─────────────────┐
│       105       │
└─────────────────┘
         │
         ▼
┌─────────────────┐
│       107       │
└─────────────────┘
         │
         ▼
┌───────────────────────────┐
│  ┌─────────────────┐       │
│  │      123        │  109  │
│  └─────────────────┘       │
└───────────────────────────┘
         │
         ▼
┌─────────────────┐
│       125       │
└─────────────────┘
```

FIG. 2

FIG. 3

FIG. 4

FIG. 5

$$X_{dt} = \sum_{k} A_{kd} \sum_{d} (Y_{tcl} * r_{\tau ck}) s_l + \epsilon_{dt}$$

61

$$J = |X_{dt} - A_{kd}(Y_{tcl} * r_{\tau ck}) s_l|_2^2$$

63

FIG. 6

EP 4 395 641 B1

$$g_{kt} = (Y_{tcl} * r_{\tau ck}) s_l \qquad X_{dt} - A_{kd}(Y_{tcl} * r_{\tau ck}) s_l \quad (71)$$

$$X_{dt} = A_{kd} g_{kt} \quad (73) \qquad A_{kd}^{\dagger} X_{dt} = g_{kt} = W_{kd} X_{dt} \quad (74)$$

$$(75) \quad J_s = \| W_{kd} X_{td} - (Y_{tcl} * r_{\tau ck}) s_l \|_2^2$$

$$(76) \quad J_s = \| W_{kd} X_{td} \|_2^2 - 2 W_{kd} X_{td} (Y_{tcl} * r_{\tau ck}) s_l + \| (Y_{tcl} * r_{\tau ck}) s_l \|_2^2$$

- - - - - - - - - - - - - - - - - - - - - - - - - - - - - - - - - - - - - - - - - - -

For $W_{kd}$ $\qquad \| W_{kd} X_{td} \|_2^2 = W_{kd} X_{td} X_{td} W_{kd}' = W_{kd} \Sigma_{dd}^{XX} W_{kd}' = I_{kk}$

For $r_{tek}$ $\qquad \| r_{\tau ck} * (Y_{tcl} s_l) \|_2^2 = r_{\tau ck} * (Y_{tcl} s_l)(s_l Y_{tcl}')* r_{\tau ck} = r_{\tau ck} \Sigma_{\tau c \tau c}^{YY s} r_{\tau ck} = I_{kk}$

For $s_l$ $\qquad \forall_l s_l \geq 0, \sum_l s_l = 1$ $\qquad \qquad \underline{81}$

- - - - - - - - - - - - - - - - - - - - - - - - - - - - - - - - - - - - - - - - - - -

$$J_{cca} = - W_{kd} X_{td} (Y_{tcl} * r_{\tau ck}) s_l \quad \text{s.t.} \quad W_{kd} \Sigma_{dd}^{XX} W_{kd}' = I_{kk}$$

$$r_{\tau ck} = r_{\tau ck} \Sigma_{\tau c \tau c}^{YY s} r_{\tau ck} = I_{kk}$$

$$\underline{83} \qquad \qquad \forall_l s_l \geq 0, \sum_l s_l = 1$$

FIG. 7

FIG. 8

$$J_{clk} = \left| X_{hk} - Y_{hk}s \right|_2^2$$

$$\text{s.t.} \quad \forall_l s_l \geq 0, \sum_l s_l = 1$$

- - - - - - - - - - - - - - - - - - - - - - - - - - - - - - - - - -

$$s(l) = \frac{1}{1+e^{-a(l-b)}}$$

$$J_{rclk} = \left| X_{hk} - Y_{hk}s \right|_2^2 + \lambda R(s_l)$$

$$\text{s.t.} \quad \forall_l s_l \geq 0, \sum_l s_l = 1$$

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6602202 B2

- US 10314508 B2

**Non-patent literature cited in the description**

- **PEGGY KORCZAK et al.** Auditory Steady-State Responses. *Journal of the American Academy of Audiology,* March 2012, vol. 23 (3), 146-70
- **MUHAMMAD AZEEM ASLAM et al.** Comparison of auditory brainstem response and auditory steady state response audiometry by evaluating the hearing thresholds obtained in children with different severity of hearing loss. *Pakistan Journal of Medical Sciences,* March 2019, vol. 35 (2), 320-324

- **CLAUS ELBERLING et al.** Auditory brainstem responses to a chirp stimulus designed from derived-band latencies in normal-hearing subjects. *J. Acoust. Soc. Am.,* November 2008, vol. 124 (5), 3022-3037
- **FELIX W. GEMBLER et al.** Five Shades of Grey: Exploring Quintary m-Sequences for More User-Friendly c-VEP-Based BCIs. *Computational Intelligence and Neuroscience,* 2020, vol. 2020, 11